# Europäisches Patentamt

# European Patent Office

# Office européen des brevets

(19)

(11) Veröffentlichungsnummer: **0 128 489**
**B1**

(12)

# EUROPÄISCHE PATENTSCHRIFT

(45) Veröffentlichungstag der Patentschrift:
29.11.89

(51) Int. Cl.⁴: **C 07 C 67/343**, C 07 C 69/757

(21) Anmeldenummer: **84106302.7**

(22) Anmeldetag: **01.06.84**

(54) Verfahren zur Herstellung von Succinylobernsteinsäuredialkylestern.

(30) Priorität: **06.06.83 DE 3320415**

(43) Veröffentlichungstag der Anmeldung:
**19.12.84 Patentblatt 84/51**

(45) Bekanntmachung des Hinweises auf die Patenterteilung:
**29.11.89 Patentblatt 89/48**

(84) Benannte Vertragsstaaten:
**BE CH DE FR GB IT LI**

(56) Entgegenhaltungen:
**US-A- 3 024 268**

**CHEMICAL ABSTRACTS, Band 83, 1975, Seite 464, Nr. 27711h, Columbus, Ohio, US; & JP - A - 75 19 738**

(73) Patentinhaber: **HOECHST AKTIENGESELLSCHAFT, Postfach 80 03 20, D-6230 Frankfurt am Main 80 (DE)**

(72) Erfinder: **Heise, Hartmut, Dr., Am Rehsteig 8, D-6232 Bad Soden am Taunus (DE)**
Erfinder: **Hintzmann, Manfred, Dr., Sachsenring 48, D-6238 Hofheim am Taunus (DE)**
Erfinder: **Brückmann, Konrad, Limburger Strasse 42e, D-6240 Köningstein/Taunus (DE)**

ACTORUM AG

## Beschreibung

Die vorliegende Erfindung betrifft ein Verfahren zur Herstellung von Succinylobernsteinsäuredialkylestern durch Kondensation von Bernsteinsäuredialkylestern bei leichter Rührbarkeit der Reaktionsmischungen unter fast völliger Vermeidung der Bildung von Dialkylethern.

Es ist bekannt, Succinylobernsteinsäuredialkylester durch Behandeln von Bernsteinsäuredialkylestern mit metallischem Natrium, Natriumalkoholaten oder Natriumamid herzustellen [Ann. 404, 272 (1914); DE-PS 1 082 907; US-PS 3 045 040; US-PS 2 821 541; DE-OS 3 104 644; DE-PS 965 403]. Als Verdünnungsmittel für die hierbei stattfindende Esterkondensation wurden bisher u.a. Diethylether (Organic Reactions, Band I (1942), Seite 283), Dioxan (DE-PS 965 403), Gemische aus Diphenyl und Diphenylether (US-PS 2 821 541), Dimethylformamid und N-Methylpyrrolidon (US-PS 3 045 040, DE-PS 1 082 907) sowie Alkohole (DE-OS 3 104 644) vorgeschlagen. Die meisten der genannten Verdünnungsmittel haben den Nachteil, daß sie sich nur sehr aufwendig regenerieren bzw. entwässern lassen, sodaß ihre Verwendung in technischem Umfang nicht gegeben ist. Hinzukommt, daß bei den in den genannten Verdünnungsmitteln durchgeführten Kondensationsreaktionen häufig sehr schlechte Rührbarkeit der Reaktionsmischung auftritt, was zu Verkrustungen an den Reaktorwänden, schlechter Durchmischung der Reaktionskomponenten und damit zu verlängerten Reaktionszeiten und unvollständigem Umsatz mit entsprechend geringeren Ausbeuten führt.

Für die Bernsteinsäuredialkylesterkondensation sind bisher vorwiegend niedermolekulare Bernsteinsäuredialkylester verwendet worden, insbesondere Bernsteinsäuredimethylester und Bernsteinsäurediethylester. Als Kondensationsmittel wurden hierbei in den meisten Fällen die Alkalimetallalkoholate der der alkoholischen Komponente der Bernsteinsäuredialkylester entsprechenden Alkohole vorgeschlagen. Hierbei hat sich als großer Nachteil erwiesen, daß bei der Kondensation des Dimethylesters bzw. Diethylesters der Bernsteinsäure in Gegenwart der entsprechenden Alkalimetallalkoholate erhebliche Mengen an Dimethylether bzw. Diethylether oder – bei Verwendung eines Alkalimetallalkoholats eines Alkohols, der von der alkoholischen Komponente des Diesters abweicht – an Mischethern entstehen, deren Entfernung einen erheblichen verfahrenstechnischen Aufwand erfordert. Als Nebenprodukte auftretende niedere Dialkylether belasten das Verfahren wegen der damit verbundenen Brand- und Explosionsgefahren; außerdem belasten sie die Umwelt, weil sich diese aus dem Abgasstrom nur schwer absorbieren und eliminieren lassen.

Es wurde nun überraschenderweise gefunden, daß man Succinylobernsteinsäuredialkylester, deren Carbonsäureestergruppen 2–6 Kohlenstoffatome enthalten, oder deren Alkalimetallsalze bei leichter Rührbarkeit der Reaktionsmischung unter weitestgehender Vermeidung der Bildung von Dialkylethern mit guten Ausbeuten durch Kondensation von Bernsteinsäuredialkylestern in Gegenwart von Alkalimetallalkoholaten in einem Verdünnungsmittel herstellen kann, indem man einen Bernsteinsäuredialkylester mit Carbonsäureestergruppen von 2 bis 6 Kohlenstoffatomen, wobei die Carbonsäureestergruppen gleich oder verschieden sein können und wobei mindestens 50% aller Carbonsäureestergruppen der zur Kondensation gelangenden Menge an Bernsteinsäuredialkylester Carbonsäureisopropylestergruppen sind, in Gegenwart eines Alkalimetallalkoholats eines gesättigten Fettalkohols von 1 bis 4 Kohlenstoffatomen in einem wasserdampflüchtigen, leicht zu entwässernden und höher als der im angewandten Alkoholat enthaltene Alkohol siedenden, mindestens jedoch bei 90 °C siedenden aromatischen Kohlenwasserstoff (Verdünnungsmittel) bei Temperaturen von 90 °C bis 150 °C kondensiert und den gebildeten Succinylobernsteinsäuredialkylester als Alkalimetallsalz in an sich bekannter Weise isoliert oder durch Zugabe einer Säure den genannten Diester freisetzt und durch Entfernen des Verdünnungsmittels in an sich bekannter Weise isoliert.

Die als Verdünnungsmittel zur Anwendung gelangenden aromatischen Kohlenwasserstoffe sollen einen Siedepunkt von etwa 90 °C bis etwa 180 °C (bei Normalbedingungen) aufweisen, mit der Maßgabe, daß sie jeweils einen höheren Siedepunkt als die im angewandten Alkoholat enthaltenen, während der Kondensation freiwerdenden Fettalkoholate von 1 bis 4 Kohlenstoffatomen haben. Außerdem sollen sie wasserdampflüchtig, leicht entwässerbar und leicht regenerierbar sein. Aromatische Kohlenwasserstoffe, welche diese Voraussetzungen erfüllen und demgemäß verfahrensgemäß eingesetzt werden können, sind beispielsweise Toluol, Xylol, Mesitylen, Ethylbenzol, Chlorbenzol, o- und m-Dichlorbenzol.

Das Alkalimetallalkoholat kann als solches oder als Lösung eingesetzt werden, wobei es im letztgenannten Fall zweckmäßig ist, den dem Alkoholat entsprechenden Alkohol als Lösungsmittel zu verwenden. Geeignete Alkalimetallalkoholate sind beispielsweise Natriummethylat, Natriumethylat, Natriumisopropylat und Kaliumisopropylat. Die genannten Isopropylate können hergestellt werden durch Umsetzung von Isopropanol mit metallischem Natrium (JACS 56, (1934), Seite 1768) oder durch Umsetzung von festem Kalium- oder Natriumhydroxid mit Isopropanol (US-PS 2 796 443).

Wendet man das Alkalimetallalkoholat gelöst in einem Fettalkohol in einem aromatischen Kohlenwasserstoff als Verdünnungsmittel der genannten Art an, so soll der betreffende als Lösungsmittel eingesetzte Fettalkohol vor der Umsetzung des Alkoholats mit dem Bernsteinsäuredialkylester aus dem Reaktionsgemisch entfernt werden. Lediglich bei der Verwendung von Isopropanol als Lösungsmittel für Alkalimetallisopropylat wird der genannte Alkohol während der Esterkondensation zusammen mit dem bei der Reaktion freiwerdenden Alkohol abdestilliert.

Es hat sich gezeigt, daß wenn mindestens 50% aller Carbonsäureestergruppen der zur Umsetzung (Kondensation) gelangenden Menge an Bernsteinsäuredialkylester Carbonsäureisopropylestergruppen sind, die Suspension der Alkalimetallsalze der Bernsteinsäuredialkylester bzw. Succinylobernsteinsäuredialkylester oder Mischungen aus beiden (mit Carbonsäureestergruppen von jeweils 2–6 Kohlenstoffatomen) in einem der genannten aromatischen Kohlenwasserstoffe (Verdünnungsmittel) fein und damit leicht rührbar bleibt. Diese leichte Rührbarkeit des Reaktionsgemisches ist von großer Bedeutung, weil dadurch Verkrustungen an den Reaktorwänden und damit verbundene schlechte Durchmischung der Reaktionskomponenten mit der Folge verlängerter Reaktionszeiten und unvollständigen Umsatzes vermieden werden.

Es hat sich ferner gezeigt, daß mit steigender Kohlenstoffatomzahl des Alkylrestes der Carbonsäurealkylestergruppen im eingesetzten Bernsteinsäuredialkylester die Neigung zur Etherbildung stark abnimmt, sodaß bei der erfindungsgemäßen Esterkondensation Diisopropylether bzw. Isopropylalkyl-Mischether nur noch in Spuren gebildet werden. Diese an sich nur noch in sehr geringen Mengen entstehenden Ether lassen sich im Gegensatz zum Dimethylether aufgrund ihres höheren Siedepunkts ohne Schwierigkeiten vom gebildeten Succinylobernsteinsäuredialkylester entfernen.

Die im Einzelfall bei der Kondensation angewandte Reaktionstemperatur richtet sich zwangsläufig nach dem Siedepunkt des im eingesetzten Alkoholat enthaltenen, während der Kondensation freiwerdenden Alkohols, da letzterer während der Kondensationsreaktion abdestilliert wird.

Die Vorteile des erfindungsgemässen Verfahrens werden nicht nur dann erreicht, wenn man einen Bernsteinsäuredialkylester mit zwei identischen Carbonsäurealkylestergruppen mit einem Alkalimetallalkoholat, dessen Alkoholkomponente die gleiche Alkylgruppe wie der Diester aufweist, umsetzt, sondern auch dann, wenn man einen Mischester oder ein Gemisch aus zwei hinsichtlich der Carbonsäureestergruppen verschiedenen Diestern umsetzt, sofern jeweils mindestens 50% aller in der zur Kondensation gelangenden Menge an Bernsteinsäuredialkylester enthaltenen Carbonsäurealkylestergruppen Carbonsäureisopropylestergruppen sind.

Man kann hierbei so vorgehen, daß man beispielsweise Bernsteinsäurediisopropylester mit einem beliebigen Alkalimetallalkoholat eines Fettalkohols von 1 bis 4 Kohlenstoffatomen in einem aromatischen Kohlenwasserstoff der weiter oben genannten Art kondensiert (Variante 1), oder daß man einen beliebigen Bernsteinsäuredialkylester (mit zwei gleichen Carbonsäurealkylestergruppen von 2–6 Kohlenstoffatomen) in Gegenwart eines Alkalimetallisopropylats in einem aromatischen Kohlenwasserstoff der genannten Art kondensiert (Variante 2).

Unbeschadet welche Variante man anwendet ist es in jedem Fall zweckmäßig, das angewandte Alkalimetallalkoholat in dem dem Alkoholat entsprechenden Fettalkohol gelöst bzw. suspendiert einzusetzen.

Bei der erstgenannten Variante erfolgt, sofern die Alkoholkomponente des angewandten Alkoholats nicht Isopropylalkohol ist, teilweise Umesterung, wobei hauptsächlich der Mischester und nur zu einem geringen Teil der dem eingesetzten Alkoholat entsprechende Dialkylester entsteht und ein entsprechender Teil des ursprünglichen Diisopropylesters erhalten bleibt. So bilden sich beispielsweise bei der Einwirkung von Natriummethylat auf Bernsteinsäurediisopropylester in Toluol 1% Dimethylester und 17% Methylisopropylester, während 82% des eingesetzten Diisopropylesters unverändert bleiben. Läßt man unter den gleichen Bedingungen Alkalimetallethylat auf Bernsteinsäurediisopropylester einwirken, so erhält man ein Estergemisch bestehend aus 1% Diethylester, 19% Ethylisopropylester und 80% unverändertem Diisopropylester bzw. die entsprechenden Alkalimetallsalze (Enolate).

Bei Variante 2 findet bereits bei Raumtemperatur eine Umesterung statt. So bilden sich bei der Einwirkung von Natriumisopropylat (suspendiert in Isopropanol) auf die nachstehend angegebenen Bernsteinsäuredialkylester folgende Estergemische bzw. deren Natriumsalze (Enolate):

| Bernsteinsäure-dialkylester (Ausgangsester) | unverändert % | Mischester % | Diisopropyl-ester % | Verdünnungs-mittel |
|---|---|---|---|---|
| Di-Methyl- | 6 | 44 | 50 | Toluol |
| Di-Ethyl- | 9 | 46 | 45 | Toluol |
| Di-n-Propyl | 12 | 43 | 45 | Xylol |
| Di-sek.-Butyl- | 11 | 45 | 44 | Xylol |
| Di-i-Butyl- | 12 | 50 | 38 | Xylol |
| Di-n-Butyl- | 22 | 47 | 31 | Xylol |

Sowohl bei Variante 1 als auch bei Variante 2 entstehen bei Raumtemperatur innerhalb kürzester Zeit vor der Kondensationsreaktion Estergemische, bei denen 50% aller vorhandenen Carbonsäurealkylestergruppen Carbonsäureisopropylestergruppen sind, weshalb die Verfahrensvarianten 1 und 2 ebensogut anwendbar sind wie diejenige Verfahrensweise, bei welcher man vom Bernsteinsäurediisopropylester unter Verwendung von Alkalimetallisopropylat ausgeht.

Bevorzugt ist die Kondensation des Bernsteinsäurediisopropylesters in Gegenwart von Natriumisopropylat (suspendiert in Isopropanol), beispielsweise in Toluol als Verdünnungsmittel bei etwa 105 °C zum Succinylobernsteinsäurediisopropylester, weil hierbei die Dialkyletherbildung (Diisopropylether) nahezu vollständig unterbleibt (< 0,1%). Hinzu kommt, daß bei der Kondensation des Diisopropylesters der Bernsteinsäure in einem aromatischen Kohlenwasserstoff (Verdünnungsmittel) der genannten Art sowohl die Suspension der Alkalimetallsalze des Diisopropylesters der Bernsteinsäure (Ausgangsester) als auch die Suspensionen der Alkalimetallsalze der gebildeten Succinylobernsteinsäure in den Verdünnungsmitteln besonders gut rührbar sind im Vergleich zu den entsprechenden Salzen von Dimethyl-, Diethyl- oder Methyl-Ethyl-Diester.

Das angewandte Molverhältnis von Bernsteinsäuredialkylester:Alkalimetallalkoholat beträgt 1:1 bis 1:1,5, vorzugsweise 1:1,1 bis 1:1,2.

Nach dem erfindungsgemäßen Verfahren läßt sich beispielsweise der Succinylobernsteinsäurediisopropylester in einer Ausbeute von 83%, bezogen auf eingesetzten Bernsteinsäurediisopropylester, herstellen, während bei der bekannten Herstellung des Succinylobernsteinsäurediisopropylesters aus γ-Halogenacetessigsäureester mittels starker Basen in einem organischen Lösungsmittel nur Ausbeuten von 68,3% bzw. 63,9% erzielt werden [DE-OS 2 317 266 und DE-OS 2 313 329].

Die Gewinnung des Succinylobernsteinsäuredialkylesters aus dem angefallenen Reaktionsgemisch erfolgt in an sich bekannter Weise, indem man die Suspension des erfindungsgemäß erhaltenen Dialkalimetallsalzes des Diesters in dem angewandten aromatischen Kohlenwasserstoff (Verdünnungsmittel) mit einem Überschuß einer organischen oder anorganischen Säure versetzt. Die hierbei anfallende Lösung des Succinylobernsteinsäuredialkylesters im aromatischen Kohlenwasserstoff kann unmittelbar weiterverarbeitet werden. Der Succinylobernsteinsäuredialkylester kann aber auch nach Entfernen des Verdünnungsmittels durch Wasserdampfdestillation aus der zurückgebliebenen wäßrigen Suspension durch Abfiltrieren in guten Ausbeuten isoliert werden.

Succinylobernsteinsäuredialkylester lassen sich in bekannter Weise in 2,5-Diarylaminoterephthalsäuren überführen, welche als Zwischenprodukte zur Herstellung von Chinacridonpigmenten von Bedeutung sind.

Beispiel 1

Ein Gemisch aus 164 g 30%iger Natriumisopropylat-Suspension in Isopropanol und 300 ml Toluol sowie 101 g Bernsteinsäurediisopropylester wird innerhalb von 3 Stunden auf 105 °C erhitzt und danach 4 Stunden bei dieser Temperatur gehalten. Während der ganzen Zeit wird Isopropanol über eine kurze Kolonne aus dem Reaktionsgemisch abdestilliert. Nahe Beendigung der Nachreaktionszeit wird die auf 90 °C gekühlte, dünnflüssige Suspension auf ein Gemisch aus 200 ml Wasser und 50 ml konz. Salzsäure gekippt. Nach Phasentrennung und Waschen mit Wasser und 2%iger Natriumhydrogencarbonat-Lösung wird die organische Phase bis zu einer Kopftemperatur von 100 °C wasserdampfdestilliert. Der aus dem Destillationsrückstand beim Erkalten sich abscheidende Ester wird auf einer Nutsche isoliert und zweimal mit je 100 ml Wasser gewaschen. Nach dem Trocknen erhält man 59 g (83% der Theorie) Succinylobernsteinsäurediisopropylester vom Schmelzpunkt 102 °C.

Beispiel 2

Aus einem Gemisch von 108 g 30%iger Natriummethylat-Lösung in Methanol und 300 ml Toluol werden ca. 85 g Methanol/Toluol abdestilliert.

Nach Zugabe von 101 g Bernsteinsäurediisopropylester wird innerhalb von 3 Stunden von Raumtemperatur auf 105 °C geheizt und 4 Stunden bei dieser Temperatur gehalten. Die Aufarbeitung des Reaktionsgemisches erfolgt wie in Beispiel 1. Die Ausbeute an Succinylobernsteinsäuredialkylester (bestehend aus 17,5% Dimethylester, 46,6% Methylisopropylester und 35,9% Diisopropylester) beträgt 51,3 g (78,6% der Theorie).

Beispiel 3

Aus einem Gemisch von 108 g 30%iger Natriummethylat-Lösung in Methanol und 300 ml Ethylbenzol werden ca. 85 g Methanol/Ethylbenzol abdestilliert. Nach Zugabe von 101 g Bernsteinsäurediisopropylester wird innerhalb von 3 Stunden von Raumtemperatur auf 125–130 °C geheizt und 4 Stunden bei dieser Temperatur gehalten. Die Aufarbeitung des Reaktionsgemisches erfolgt wie in Beispiel 1. Die Ausbeute an Succinylobernsteinsäuredialkylester (bestehend aus 23,7% Dimethylester, 50,0% Methylisopropylester und 26,3% Diisopropylester) beträgt 51,0 g (79,5% der Theorie).

Beispiel 4

Aus einem Gemisch von 108 g 30%iger Natriummethylat-Lösung in Methanol und 300 ml Xylol werden ca. 75–80 g Methanol/Xylol abdestilliert. Nach Zugabe von 101 g Bernsteinsäurediisopropylester wird weitergearbeitet wie in Beispiel 3 beschrieben. Die Ausbeute an Succinylobernsteinsäuredialkylester (bestehend aus 24,0% Dimethylester, 48,0% Methylisopropylester und 28,0% Diisopropylester) beträgt 49,5 g (77,0% der Theorie).

Beispiel 5

Ein Gemisch aus 164 g 30%iger Natriumisopropylat-Suspension in Isopropanol, 300 ml Toluol und 73 g Bernsteinsäuredimethylester wird innerhalb von 3 Stunden auf 105 °C erhitzt. Bei dieser Temperatur wird 4 Stunden gerührt, wobei der Alkohol über eine kurze Kolonne abdestilliert. Anschließend wird die auf 90 °C gekühlte dünne Suspension auf ein Gemisch aus 200 ml Wasser und 50 ml konz. Salzsäure gegeben. Nach Phasentrennung und Waschen mit Wasser und 2%iger Natriumhydrogencarbonat-Lösung wird die organische Phase ohne Zwischenisolierung des Succinylobernsteinsäuredialkylesters mit Anilin zu Dianilinodihydroterephthalsäureester umgesetzt.

Der Gehalt an Succinylobernsteinsäuredialkylester (bestehend aus 15,8% Dimethylester, 48,7% Methylisopropylester und 35,5% Diisopropylester) in der organischen Phase entspricht einer Ausbeute von 75,4% der Theorie.

Beispiel 6

Nach dem in Beispiel 5 beschriebenen Verfahren erhält man bei Einsatz von 87 g Bernsteinsäurediethylester anstelle von 73 g Bernsteinsäuredimethylester eine Ausbeute an Succinylobernsteinsäuredialkylester (bestehend aus 16,5% Diethylester, 44,0% Ethylisopropylester und 39,5% Diisopropylester) von 53,8 g (78,7% der Theorie).

Beispiel 7

Ein Gemisch aus 164 g 30%iger Natriumisopropylat-Suspension in Isopropanol, 300 ml Xylol und 115 g Bernsteinsäuredi-sek-butylester wird langsam auf 130°C erhitzt und 3 Stunden bei dieser Temperatur gehalten. Während der ganzen Zeit wird Isopropanol bzw. bei der Kondensation freiwerdendes sek-Butanol aus dem Reaktionsgemisch abdestilliert. Die Aufarbeitung erfolgt wie in Beispiel 1 beschrieben.

Die Ausbeute an Succinylobernsteinsäuredialkylester (bestehend aus 39,6% Diisopropylester, 44,2% Isopropyl-sek-butylester und 16,2% Di-sek-butylester) beträgt 61,9 g (84,1% der Theorie).

Beispiel 8

Analog Beispiel 7 wird ein Gemisch aus 164 g 30%iger Natriumisopropylat-Suspension in Isopropanol, 300 ml Xylol und 115 g Bernsteinsäurediisobutylester langsam unter Abdestillieren von Alkohol auf 120°C erhitzt und 3 Stunden bei dieser Temperatur gehalten. Die Ausbeute an Succinylobernsteinsäureester (bestehend aus 19,2% Diisopropylester, 45,6% Isopropylisobutylester und 35,2% Diisobutylester) beträgt 61,2 g (81,6% der Theorie).

Beispiel 9

Unter den gleichen Bedingungen wie in Beispiel 8 erhält man beim Einsatz von Bernsteinsäuredi-n-butylester eine Ausbeute an Succinylobernsteinsäuredialkylester (bestehend aus 21,3% Diisopropylester, 46,5% Isopropyl-n-butylester und 32,2% Di-n-butylester) von 59,1 g (79,0% der Theorie).

Beispiel 10

Bei der Kondensation von 101 g Bernsteinsäuredi-n-propylester unter den Bedingungen von Beispiel 8 erhält man eine Ausbeute an Succinylobernsteinsäurepropylester von 59,7 g (84,0% der Theorie).

Beispiel 11

Aus einem Gemisch von 136 g 30%iger Natrium-ethylat-Lösung in Ethanol und 300 ml Toluol werden ca. 90–95 g Ethanol/Toluol abdestilliert. Nach Abkühlen und Zugabe von 101 g Bernsteinsäurediisopropylester erhält man bei der Kondensation analog Beispiel 2 eine Ausbeute an Succinylobernsteinsäuredialkylester (bestehend aus 16,6% Diethylester, 46,7% Ethylisopropylester und 36,7% Diisopropylester) von 51,9 g (76,1% der Theorie).

Beispiel 12

Aus einem Gemisch von 108 g 30%iger Natriummethylat-Lösung in Methanol und 300 ml Toluol werden ca. 85 g Methanol/Toluol abdestilliert.

Nach Zugabe von 150 g Isopropanol und 73 g Bernsteinsäuredimethylester wird innerhalb von 5 Stunden von Raumtemperatur auf 105°C geheizt und 3 Stunden bei dieser Temperatur gehalten. Während der ganzen Zeit wird Methanol/Isopropanol über eine kurze Kolonne aus dem Reaktionsgemisch abdestilliert. Die Aufarbeitung erfolgt entsprechend Beispiel 1. Die Ausbeute an Succinylobernsteinsäuredialkylester (bestehend aus 22,3% Dimethylester, 47,1% Methylisopropylester und 30,6% Diisopropylester) beträgt 49,3 g (76,3% der Theorie).

Beispiel 13

Der entsprechend Beispiel 2 hergestellte Succinylobernsteinsäuredialkylester wird, wie in Beispiel 5 beschrieben, ohne Zwischenisolierung mit 93 g Anilin und 30 g Eisessig bei 110 bis 115°C zu Dianilinodihydroterephthalsäureester umgesetzt. Nach Luftoxidation bei 70°C wird der Dianilinoterephthalsäureester mit 156 g Natronlauge (32%ig) bei 75–80°C in 3 Stunden verseift. Nach anschließender Wasserdampfdestillation und Klärung wird mit Salzsäure angesäuert, das Produkt auf einer Nutsche isoliert, mit verdünnter Salzsäure und Wasser gewaschen und getrocknet. Die Ausbeute an Dianilinoterephthalsäure beträgt 61,9 g (71,1% der Theorie).

**Patentansprüche**

1. Verfahren zur Herstellung von Succinylobernsteinsäuredialkylestern, deren Carbonsäureestergruppen 2–6 Kohlenstoffatome enthalten, oder deren Alkalimetallsalzen bei leichter Rührbarkeit der Reaktionsmischung unter weitestgehender Vermeidung der Bildung von Dialkylethern mit guten Ausbeuten durch Kondensation von Bernsteinsäuredialkylestern in Gegenwart von Alkalimetallalkoholaten in einem Verdünnungsmittel, dadurch gekennzeichnet, daß man einen Bernsteinsäuredialkylester mit Carbonsäureestergruppen von 2 bis 6 Kohlenstoffatomen, wobei diese Estergruppen gleich oder verschieden sein können und wobei mindestens 50% aller Carbonsäureestergruppen der zur Kondensation gelangenden Menge an Bernsteinsäuredialkylester Carbonsäureisopropylestergruppen sind, in Gegenwart eines Alkalimetallalkoholats eines gesättigten Fettalkohols von 1–4 Kohlenstoffatomen in einem wasserdampfflüchtigen, leicht zu entwässernden und höher als der im angewandten Alkoholat enthaltene Alkohol siedenden, mindestens jedoch bei 90 °C siedenden aromatischen Kohlenwasserstoff (Verdünnungsmittel) bei Temperaturen von 90° bis 150 °C kondensiert und den gebildeten Succinylobernsteinsäuredialkylester als Alkalimetallsalz in an sich bekannter Weise isoliert oder durch Zugabe einer Säure den genannten Diester freisetzt und durch Entfernen des Verdünnungsmittels in an sich bekannter Weise isoliert.

2. Verfahren nach Anspruch 1, dadurch gekennzeichnet, daß man die Kondensation in Toluol, Xylol, Mesitylen, Ethylbenzol, Chlorbenzol, o- oder m-Dichlorbenzol als Verdünnungsmittel durchführt.

3. Verfahren nach Anspruch 1 oder 2, dadurch gekennzeichnet, daß man die Kondensation in Gegenwart von Natrium- oder Kaliummethylat, -ethylat oder -iso-propylat durchführt.

## Claims

1. A process for the preparation of a dialkyl succinosuccinate, the carboxylic acid ester groups of which contain 2–6 carbon atoms, or an alkali metal salt thereof with good yields by condensation of a dialkyl succinate in the presence of an alkali metal alcoholate in a diluent under conditions under which the reaction mixture can easily be stirred and the formation of the dialkyl ether is as far as possible avoided, which comprises condensing a dialkylic succinate with carboxylic acid ester groups of 2 to 6 carbon atoms, it being possible for these ester groups to be identical or different and at least 50% of all the carboxylic acid ester groups of the amount of dialkyl succinate undergoing condensation being isopropyl carboxylate groups, in the presence of an alkali metal alcoholate of a saturated fatty alcohol having 1–4 carbon atoms at temperatures of 90 °C to 150 °C in an aromatic hydrocarbon (diluent) which is volatile in steam, is easy to dehydrate and has a boiling point above that of the alcohol contained in the alcoholate used, but of at least 90 °C, and isolating the resulting dialkyl succinosuccinate as the alkali metal salt in a manner which is known per se, or liberating the diester mentioned by addition of an acid and isolating it by removing the diluent in a manner which is known per se.

2. The process as claimed in claim 1, wherein the condensation is carried out in toluene, xylene, mesitylene, ethylbenzene, chlorobenzene or o- or m-dichlorobenzene as the diluent.

3. The process as claimed in either of claims 1 and 2, wherein the condensation is carried out in the presence of sodium methylate, ethylate or iso-propylate or potassium methylate, ethylate or iso-propylate.

## Revendications

1. Procédé pour préparer, avec de bons rendements, des succinyl-succinates de dialkyles dont les radicaux d'esters carboxyliques contiennent de 2 à 6 atomes de carbone, ou leurs sels de métaux alcalins, par condensation de succinates de dialkyles, dans un diluant, en présence d'alcoolates de métaux alcalins, procédé dans lequel le mélange réactionnel est facile à agiter et la formation d'oxydes de dialkyles est évitée dans une très large mesure et qui est caractérisé en ce qu'on condense à une température de 90 à 150 °C un succinate de dialkyle dont les radicaux d'esters carboxyliques contiennent de 2 à 6 atomes de carbone, ces radicaux d'esters étant identiques ou différents et au moins 50% de tous les radicaux d'esters carboxyliques de la quantité de succinate de dialkyle mise en jeu étant des radicaux d'esters isopropyliques, en présence d'un alcoolate de métal alcalin d'un alcool gras saturé contenant de 1 à 4 atomes de carbone, dans un hydrocarbure aromatique (diluant) entraînable à la vapeur d'eau, facile à déshydrater et bouillant à une température supérieure à la température d'ébullition de l'alcool contenu dans l'alcoolate mis en jeu mais au moins égale à 90 °C, et on isole de manière connue, à l'état de sel de métal alcalin, le succinyl-succinate de dialkyle formé, ou on libère le diester cité, par addition d'un acide, et on l'isole de manière connue par élimination du diluant.

2. Procédé selon la revendication 1 caractérisé en ce qu'on effectue la condensation en utilisant, comme diluant, le toluène, un xylène, le mésitylène, l'éthylbenzène, le chlorobenzène, l'o-dichlorobenzène ou le m-dichlorobenzène.

3. Procédé selon l'une des revendications 1 et 2, caractérisé en ce qu'on effectue la condensation en présence de méthylate, d'éthylate ou d'isopropylate de sodium ou de potassium.